# EUROPÄISCHE PATENTSCHRIFT

(11) **EP 0 582 124 B1**
(45) Veröffentlichungstag und Bekanntmachung des Hinweises auf die Patenterteilung: **11.06.1997**
(21) Anmeldenummer: 93111597.6
(22) Anmeldetag: 20.07.1993
(51) Int. Cl.: A61M 15/00, B05B 7/00, A61M 11/06, A61M 16/10, B05B 7/16, H05B 3/14

(54) **Zerstäubervorrichtung mit Heizeinrichtung**
Atomizer device with heating element
Dispositif d'atomiseur avec un élément de chauffage

(30) Priorität: 05.08.1992 DE 4225928
(43) Veröffentlichungstag der Anmeldung: 09.02.1994
(73) Patentinhaber: PAUL RITZAU PARI-WERK GmbH, D-82317 Starnberg (DE)
(72) Erfinder: Knoch, Martin Dr., 82335 Berg (DE)
(74) Vertreter: Ritter und Edler von Fischern, Bernhard,Dipl.-Ing.

(56) Entgegenhaltungen:
- EP-A- 0 170 715
- EP-A- 0 320 966
- EP-A- 0 379 873
- WO-A-85/00112
- DE-A- 3 043 537
- DE-U- 1 898 032

## Beschreibung

Die vorliegende Erfindung betrifft eine Vorrichtung zum Zerstäuben, Verteilen und Vermischen von flüssigen oder pulverförmigen Stoffen mittels eines Druckgasstromes, insbesondere für die Erzeugung von Aerosolen für Inhalationszwecke gemäß dem Oberbegriff des Patentanspruchs 1.

Eine derartige Vorrichtung ist bekannt aus dem deutschen Gebrauchsmuster DE-U-1 898 032, das eine Vorrichtung zum Zerstäuben, Verteilen und Vermischen von flüssigen und pulverförmigen Stoffen offenbart, die eine Zerstäubungsdüse in einem Zerstäubungsraum, einen Zuluftkamin für den Eintritt von Zuluft in den Zerstäubungsraum und eine Heizeinrichtung zum Erwärmen der durch den Zuluftkamin angesaugten Umgebungsluft aufweist. Die Heizeinrichtung ist auf den Zuluftkanal aufgesteckt und in Form einer Glühbirne ausgebildet, die mit ihrer Längsachse zentral in einem Rohr angeordnet ist, so daß sich ein kreisförmiger Querschnitt für die Zuluft bildet. Ferner ist in dem Gebrauchsmuster eine Heizeinrichtung zum Erwärmen des Zusatzgases bei Verneblern beschrieben, die aus einem Rohrstück besteht, welches mit seinem unteren Ende in dem Ansaugkanal eingesteckt ist und an seinem freien Ende einen elektrischen Heizdraht trägt, der entlang der inneren Mantelfläche des Rohres geführt ist.

Bei dieser Ausgestaltung ist das Größenverhältnis zwischen Vernebler und Heizeinrichtung ungünstig, denn Vernebler und Heizeinrichtung sind nahezu gleich groß. Außerdem ist die Anordnung der Heizeinrichtung am oberen Ende des Ansaugkanals aus Stabilitätsgründen ungünstig, insbesondere da der Schwerpunkt der Heizeinrichtung von der Befestigungsstelle weit entfernt ist. Da auch der Schwerpunkt des Verneblers in der Nähe des von der Zuluftkaminöffnung entgegengesetzten Endes liegt, besteht zwischen den beiden Schwerpunkten ein erheblicher Abstand, so daß das Gesamtgebilde aus Vernebler und Heizeinrichtung unhandlich und instabil ist.

Die Handhabbarkeit von Verneblern stellt mittlerweile aber einen wesentlichen Funktionalitätsaspekt dieser Therapiegeräte dar, denn neben der therapiegerechten Herstellung und Zuführung des Aerosols wird in zunehmendem Maße verlangt, daß diese Geräte einfach zu bedienen, klein und problemlos zu transportieren sind. Diese Anforderungen sind bei den bekannten Verneblern mit Heizeinrichtung nicht ausreichend erfüllt.

Der Erfindung liegt daher die Aufgabe zugrunde, eine Vorrichtung der eingangs geschilderten Art derart auszugestalten, daß ein kompakter, betriebssicher und leicht handhabbarer Aufbau vorliegt.

Gelöst wird diese Aufgabe, bei der die Heizeinrichtung im Zuluftkamin angeordnet ist und einen Wärmetauscher aus einem zylindrischen Kernkörper mit mehreren, insbesondere drei bis acht Rippen, die radial zum zylindrischen Kernkörper verlaufen, aufweist.

Vorteilhafte Ausgestaltungen ergeben sich aus den Unteransprüchen 2 bis 8.

Aus DE-A1-30 43 537 ist ein Zerstäuber bekannt, bei dem mit Hilfe eines elektrischen Heizelements nämlich einem PTC-Thermistor die zu zerstäubende Substanz erwärmt wird.

Der Grundgedanke der Erfindung besteht darin, die Erwärmung des Aerosols über den Umweg einer Erwärmung der Zuluft zu erreichen. Insbesondere bei Inhalationsgeräten mit Zuluftkamin kann eine ausreichende Erwärmung der Zuluft kontrolliert erfolgen, da wegen des Zuluftkamins eine gewisse Führung und Kanalisierung der Zuluft erfolgt. Durch Anordnung einer geeignet geformten Heizeinrichtung im Zuluftkamin wird eine Beeinträchtigung der Störmungsverhältnisse weitgehend vermieden. Insbesondere die Verteilung der Tröpfchengröße wird durch Mischung von zerstäubter Substanz und Zuluft nicht beeinträchtigt.

Vielmehr tritt durch die erfindungsgemäße Lösung ein positiver Effekt dahingehend ein, daß das Primäraerosol im Bereich der Zerstäubungsdüse stärker verdunstet wird, da die Zuluft eine höhere Temperatur aufweist. Daraus folgt eine Konzentrationszunahme in den Tröpfchen, womit bei gleichem vom Luftstrom transportierten Tröpfchenspektrum ein höherer Durchsatz der zu zerstäubenden Substanz erreicht wird. Es wurde erkannt, daß eine Beheizung des Aerosols, d.h. des Zuluft-Aerosol-Gemisches etwa im Bereich des Auslaßstutzens zu stärkeren Ablagerungen der zerstäubten Substanz und damit zu einer Reduzierung des Durchsatzes führt. Die bei einer unmittelbaren Beheizung des Aerosols auftretenden Reinigungsprobleme werden bei der erfindungsgemäßen Lösung ebenfalls vermieden.

Eine Überhitzung der zu zerstäubenden Substanz wird auf jeden Fall ausgeschlossen, da die Erwärmung der Substanz nur durch den Kontakt mit der erwärmten Zuluft erfolgt. Die Zuluft wird aber in keinem Fall über eine Temperatur von ca. 50° erwärmt.

Im folgenden wird die Erfindung anhand von Ausführungsbeispielen unter Bezugnahme auf die beiliegenden Zeichnungen genauer beschrieben, in denen zeigt:
- Fig. 1: ein bekanntes Inhalationsgerät mit Zuluftkamin,
- Fig. 2: eine erfindungsgemäße Heizeinrichtung in perspektivischer Darstellung,
- Fig. 3: einen Teil eines erfindungsgemäßen Inhalationsgeräts, das mit einer Heizeinrichtung gem. Fig. 2 ausgestattet ist, und
- Fig. 4: ein erfindungsgemäßes Inhalationsgerät, bei dem die Heizeinrichtung in einem speziell ausgestalteten Zuluftkaminkörper integriert ist.

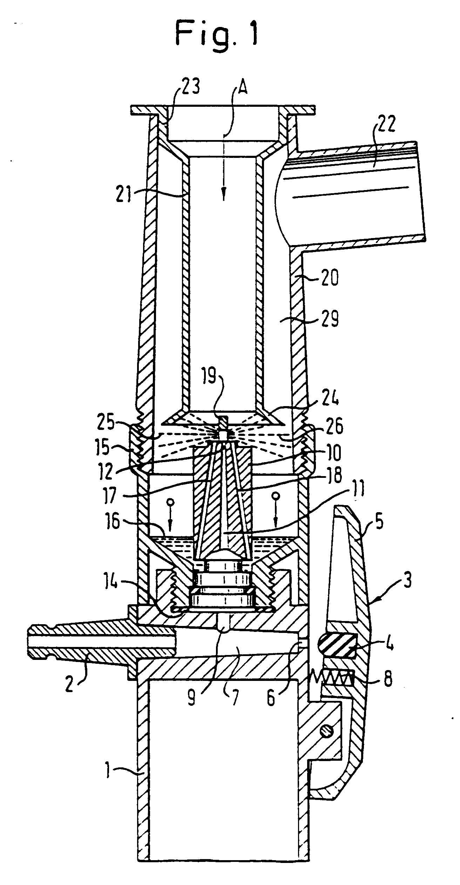

Anhand von Fig. 1 soll einleitend ein aus EP-A1-0 170 715 bekanntes Inhalationsgerät für die Behandlung der Lunge und Atemwege beschrieben werden, bei dem die erfindungsgemäße Heizeinrichtung vorgesehen werden kann und das als Beispiel für eine Zerstäubervorrichtung dient.

Das in Fig. 1 gezeigte Inhalationsgerät besteht im wesentlichen aus dem zylindrischen Verneblerunterteil 1 mit Anschlußstutzen 2 für die Druckgasleitung und dem gegenüber dem Anschlußstutzen 2 angeordneten Tasthebel 3 mit einem Dichtungseinsatz 4 zum Verschließen der Auslaßöffnung 6 im Zuleitungskanal. Der Tasthebel 3 kann vom Benutzer in einfacher Weise aus seiner in Fig. 1 gezeigten Außerbetriebstellung unter Überwindung der Kraft der Feder 8 mit dem Vorderteil des Tasthebels 3 in Anlage zur Oberfläche des Verneblers gebracht werden, wobei der Dichteinsatz 4 sich auf die Auslaßöffnung 6 legt und so den Zuleitungskanal für das Druckgas absperrt und dieses durch die Querbohrung 9 in die zentrale Druckgasleitung 11 des Düsenkopfes 10 umleitet.

An dem Verneblerunterteil 1 ist in bekannter Weise unter Zwischenschaltung eines elastischen Dichtungsringes 14, der gleichfalls zylindrische Behälter 15 zur Aufnahme der zu zerstäubenden Substanz 16 befestigt, zum Beispiel eingeschraubt, wie in Fig. 1 dargestellt. Im Behälter 15 ist auch die Zerstäuberdüse untergebracht, die aus dem Düsenkopf 10 mit der zentralen Druckgasleitung 11 und den beiden seitlichen Ansaugkanälen 17 und 18 für die zu zerstäubende Substanz 16 und dem Gasstromsteuer 19 besteht. Die zentrale Druckgasleitung 11 verjüngt sich gegen das obere Ende des Düsenkopfes und endet in der schmalen Düsenbohrung 12, die unterhalb des Gasstromsteuers 19 und diesem gegenüberliegend aus dem Düsenkopf 10 ausmündet.

An dem Behälter 15 ist die zylindrische Verneblerhaube 20 befestigt, zum Beispiel aufgeschraubt, wie in Fig. 1 dargestellt. Die Verneblerhaube 20 enthält einen koaxialen Zuluftkamin 21, der sich bis dicht über das Gasstromsteuer 19 in den Innenraum des Inhalationsgerätes hinein erstreckt. Im oberen Teil ist an die Verneblerhaube 20 der Aerosolaustrittsstutzen 22 angeformt, an den ein in der Zeichnung nicht dargestelltes Mundstück ansetzbar ist. Durch den Aerosolaustrittsstutzen 22 wird das von der Zerstäuberdüse erzeugte Aerosol vom Patienten angesaugt, wobei Umgebungsluft durch den Zuluftkamin in Richtung des Pfeiles A in das Innere des Inhalationsgerätes gelangt.

Der Zuluftkamin 21 trägt an seinem unteren Ende einen Prallschirm 24, an dem zumindest ein Teil der größeren Partikel des an den Schrägflächen des Gasstromsteuers 19 erzeugten Aerosols weiter zerstäubt wird. Der Prallschirm 24 hat ferner die Wirkung, daß zu große Partikel abgeschieden, im Behälter 15 gesammelt und einer erneuten Zerstäubung zugeführt werden.

An den Anschlußstutzen 2 wird eine Druckgasquelle (z.B. ein Kompressor), die den erforderlichen Druck von mindestens 0,6 bar erzeugt, angeschlossen. Das Druckgas strömt, wenn durch Betätigung des Tasthebels 3 die Auslaßöffnung des Zuleitungskanals 7 verschlossen wird, durch die zentrale Druckgasleitung 11 und die Düsenbohrung 12 bis zur Austrittsöffnung im Düsenkopf 10. Dabei wird durch die benachbarten Ansaugkanäle 17 und 18 die zu zerstäubende Substanz 16 aus dem unteren Bereich des Behälters 15 angesaugt und an den schräg gestellten Prallflächen des Gasstromsteuers 19 zerstäubt und verteilt. Der übliche Keilwinkel des Gasstromsteuers 19 beträgt ungefähr 120°, so daß sich beidseitig des Gasstromsteuers 19 je ein Aerosolfächer 25 und 26 über einen Winkelbereich von etwa 30° ausbildet.

Wie bereits eingangs erwähnt, tritt bei der Zerstäubung von Flüssigkeiten durch die Verdunstung insbesondere kleinster Tröpfchen eine Abkühlung ein, die dazu führt, daß die Temperatur des gesamten Aerosols stark absinkt.

In der Einatemphase wird das auf diese Weise erzeugte Aerosol durch den Aerosolauslaßstutzen 22 vom Patienten angesaugt und mit der durch den Zuluftkamin 21 zugeführten Luft vermischt.

Bei dem in Fig. 1 gezeigten Inhalationsgerät ist die Verneblerhaube 20 am oberen Ende offen und wird durch den Zuluftkamin 21 abgeschlossen, der dazu an dem dem Prallschirm 24 gegenüberliegenden Ende konisch in eine Steckkappe 23 übergeht, die an den Durchmesser der Öffnung in der Verneblerhaube 20 angepaßt ist und für einen sicheren und im wesentlichen gasdichten Sitz des Zuluftkamins sorgt.

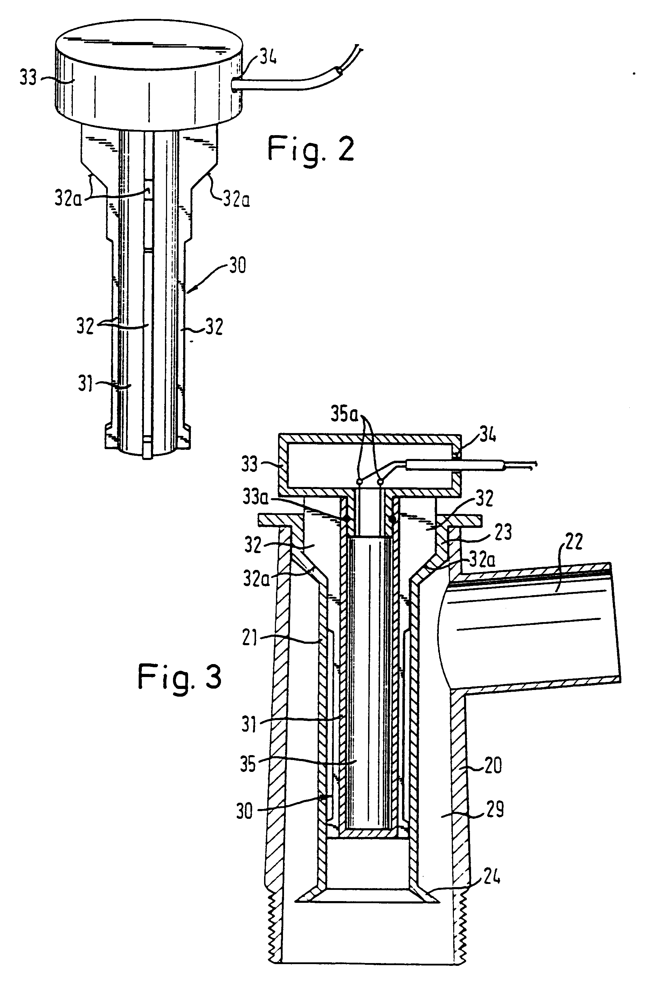

In Fig. 2 ist in einer perspektivischen Ansicht eine Heizeinrichtung 30 dargestellt, die erfindungsgemäß zur Aufheizung der durch den Zuluftkamin zugeführten Umgebungsluft dient. Die Heizeinrichtung 30 besteht aus einem zylindrischen Kernkörper 31 und mehreren Rippen 32 die sich radial vom Kernkörper weg erstrecken. Der Kernkörper 31 und die Rippen 32 sind aus einem gut wärmeleitendem Material, vorzugsweise Aluminium und einstückig hergestellt. Der Kernkörper 31 ist dünnwandig und am unteren Ende verschlossen, so daß er im Inneren ein elektrisches Heizelement aufnehmen kann. Ein Abschlußkörper 33 verschließt den Kernkörper 31 an seinem oberen Ende. Der Abschlußkörper 33 hat die Form einer flachen zylindrischen Scheibe, deren Zylinderachse koaxial zur Längsachse des Kernkörpers 31 verläuft. Der Durchmesser des Abschlußkörpers ist so groß, daß der Abschlußkörper den aus dem Kernkörper und den Rippen bestehenden Wärmetauscherteil der Heizeinrichtung allseitig überragt. Dadurch kann der Abschlußkörper 33 auch als Griffteil dienen, an dem die Heizeinrichtung 30 gefaßt werden kann. Seitlich ist am Abschlußkörper eine Öffnung 34 vorgesehen, durch die die elektrischen Versorgungsleitungen für das elektrische Heizelement geführt werden.

Die Rippen 32 der Heizeinrichtung 30 sind so geformt, daß schräg verlaufende Auflageflächen 32a gebildet werden, auf denen die Heizeinrichtung 30 aufliegt, wenn sie in den Zuluftkamin des bekannten Inhalationsgerätes eingesetzt ist, wie in Fig. 3 dargestellt ist. Der Verlauf der Auflageflächen 32a entspricht im wesentlichen dem konischen Teil der Steckkappe 23 des Zuluftkamins.

Die Auflageflächen 32a der Rippen 32 gehen jeweils in einen Abschnitt über, in dem die Rippen 32 eine konstante Höhe haben, die so gewählt ist, daß der Abstand der Aussenflächen dem Durchmesser des Zuluftkamins entspricht. An diesen Abschnitt schließt sich ein Abschnitt an, in dem die Rippen eine geringere Höhe aufweisen, so daß zwischen der Aussenfläche der Rippen und dem Zuluftkamin ein Spalt entsteht, wie in Fig. 3 erkennbar ist. Am unteren Ende besitzen die Rippen 32 erneut einen kurzen Abschnitt, in dem die Höhe der Rippen so gewählt ist, daß die äusseren Oberflächen der Rippen einen Abstand zueinander besitzen, der dem Durchmesser des Zuluftkamins 21 entspricht.

In Fig. 3 ist nur die Verneblerkappe 20 des in Fig. 1 gezeigten Inhalationsgerätes, sowie der darin eingesetzte Zuluftkamin 21 dargestellt. In den Zuluftkamin ist die erfindungsgemäße Heizeinrichtung eingesetzt, so daß der Wärmetauscher mit den Auflageflächen 32a an dem konischen Teil der Steckkappe 23 des Zuluftkamins anliegt. Der Wärmetauscher erstreckt sich im Zuluftkamin 21 bis in die Nähe des Prallschirms 24, d.h. bis in die unmittelbare Nähe der Zerstäuberdüse. Ferner ragt der Wärmetauscher nach oben aus dem Zuluftkamin heraus. Dadurch kann Umgebungsluft an den Rippen 32 vorbei durch den Zuluftkamin 21 strömen, wenn der Patient durch den Aerosolauslaßstutzen 22 das Aerosol ansaugt. Da die Umgebungsluft an dem Wärmetauscherteil entlang strömt, wird die Umgebungsluft erwärmt, so daß am unteren Ende des Zuluftkamins 21 bereits erwärmte Umgebungsluft für die Durchmischung mit der zerstäubten Substanz bereitsteht.

In der geschnittenen Darstellung der Fig. 3 ist zu erkennen, daß im Inneren des Wärmetauschers ein elektrisches Heizelement 35 vorgesehen ist, das an einem Ende am verschlossenen Ende des Kernkörpers 31 anliegt und das am anderen Ende vom Abschlußkörper 33 eingeklemmt wird. Der Abschlußkörper 33 besitzt dazu ein zylindrisches Einsteckteil, dessen Durchmesser so gewählt ist, daß er in die zylindrische Öffnung des Kernkörpers 31 einsteckbar ist. Ein fester Sitz wird entweder durch geeignete Wahl des Durchmessers des Einsteckteils oder durch das Einbringen eines elastischen Dichtringes 33a zwischen Einsteckteil und Kernkörper gewährleistet. Am Einsteckteil ist dazu eine umlaufende Nut vorgesehen, in die der Dichtring eingepaßt ist, wie in Fig. 3 dargestellt ist.

Der Abschlußkörper 33 und das Einsteckteil sind entweder hohl ausgebildet oder besitzen zumindest einen Durchgang, der eine Heranführung der elektrischen Versorgungsleitungen von der Öffnung 34 an die elektrischen Anschlüsse 35a des elektrischen Heizelements 35 ermöglicht.

Da der Abschlußkörper 33 allseitig den Wärmetauscher der Heizeinrichtung 30 überragt, bildet sich zwischen dem oberen Ende des Zuluftkamins und der Unterfläche des Abschlußkörpers 33 im eingesteckten Zustand ein Spalt, durch den die Umgebungsluft in den Zuluftkamin gelangen kann. Da die Rippen 32 des Wärmetauschers in Bezug auf den Abschlußkörper 33 nach innen versetzt sind, wie sich aus Fig. 3 ergibt, ist eine unbeabsichtigte Berührung der erhitzten Rippen auch in dem aus dem Zuluftkamin herausragenden Bereich ausgeschlossen.

Bei dem elektrischen Heizelement 35 handelt es sich vorzugsweise um einen PTC-Widerstand (Kaltleiter), dessen elektrischer Widerstand mit steigender Temperatur abnimmt. Man kann daher einen PTC-Widerstand und die ihn speisende Spannungsquelle so aufeinander abstimmen, daß eine bestimmte Betriebstemperatur selbstregelnd erreicht wird. Um eine Aerosoltemperatur am Aerosolauslaßstutzen 22 von ca. 28°C bis 32°C zu erreichen, sollte die Temperatur des elektrischen PTC-Heizelements zwischen 150°C und 170°C liegen und eine Leistungsaufnahme von ca. 10 bis 15 Watt durch den PTC-Heizwiderstand erfolgen. An der Oberfläche des Wärmetauschers treten dann Temperaturen zwischen 130°C und 150°C auf, so daß eine Erwärmung der Umgebungsluft auf die angestrebten Werte ermöglicht wird. Höhere Aerosoltemperaturen für die Therapie der oberen Atemwege lassen sich durch leistungsstärkere Heizelemente realisieren bei Temperaturen bis 220°C und Leistungsaufnahmen bis 36 Watt.

Die Temperatur des Aerosols sollte in einem Bereich zwischen 25°C und 40°C, bevorzugt zwischen 28°C und 37°C liegen.

Als Spannungsversorgung für den elektrischen PTC-Heizwiderstand ist ein herkömmliches Steckernetzteil mit einer Ausgangsspannung in der Größenordnung von ca. 12 Volt ausreichend.

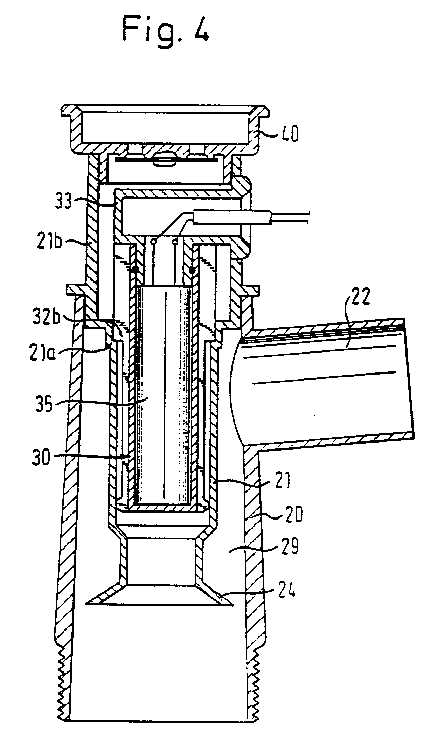

In Fig. 4 ist ein weiteres Ausführungsbeispiel dargestellt, bei dem die Heizeinrichtung 30 fest mit dem Zuluftkamin 21 verbunden ist. Die Heizeinrichtung besteht auch bei diesem Ausführungsbeispiel aus einem wärmetauscherteil und einem darin angeordneten elektrischen Heizelement 35, wobei der Wärmetauscherteil aus einem zylindrischen Kernkörper und sich radial erstreckenden Rippen besteht. Jedoch weisen die Rippen keine schräg verlaufenden Auflageflächen auf, da der Zuluftkamin an die Heizeinrichtung angepaßt ist.

Wie in Fig. 4 gezeigt, liegen die Rippen des Wärmetauschers mit einer vorspringenden Kante 32b auf einem Absatz 21a auf, der an der Innenwand des Zuluftkamins 21 umlaufend vorgesehen ist. Die Höhe der Rippen des Wärmetauschers ist so gewählt, daß die äusseren Flächen der Rippen einen Abstand zueinander besitzen, der dem Durchmesser des Zuluftkamins 21 unmittelbar oberhalb des Absatzes entspricht. Im weiteren Verlauf zum verschlossenen Ende des Kernkörpers hin besitzen die Rippen eine Höhe, die geringer ist, so daß die Aussenflächen der Rippen nicht am Zuluftkamin anliegen. Erst am Ende des Wärmetauschers ist die Höhe der Rippen etwas vergrößert, so daß die Aussenflächen der Rippen in diesem Bereich wieder an der Innenwand des Zuluftkamins anliegen, wie in Fig. 4 erkennbar ist. Der Wärmetauscher ist somit im Zuluftkamin sicher fixiert.

Im Gegensatz zum herkömmlichen Zuluftkamin schließt sich an das obere Ende ein zylindrischer Teil 21b an, in den sich der Wärmetauscher der Heizeinrichtung 30 erstreckt. Jedoch überragt der zylindrische Teil 21b den Wärmetauscher, so daß ein unbeabsichtigtes Berühren des aufgeheizten Wärmetauschers nicht möglich ist. Damit die Umgebungsluft durch den zylindrischen Teil hindurch und an den Rippen des Wärmetauschers vorbei in den Zuluftkamin 21 strömen kann, ist der Abschlußkörper 33 der Heizeinrichtung bei diesem Ausführungsbeispiel mit einem kleineren Durchmesser ausgestattet. Im wesentlichen entspricht der Durchmesser des Abschlußkörpers dem Abstand der Aussenflächen einander gegenüberliegender Rippen des Wärmetauschers. Dadurch wird gewährleistet, daß über die obere Öffnung der Wärmetauscher nicht berührt werden kann, aber dennoch eine ausreichende Zufuhr von Umgebungsluft für die Einatemphase gewährleistet ist. Da Zuluftkamin 21 und Heizeinrichtung 30 bei diesem Ausführungsbeispiel als Einheit aufgebaut sind, steht der Abschlußkörper 33 mit dem zylindrischen Teil 21b in Verbindung, der den Zuluftkamin 21 am oberen Ende fortsetzt. Die Zuführung der elektrischen Verbindungsleitungen erfolgt durch den aussen liegenden zylindrischen Teil 21b des Zuluftkamins und den Abschlußkörper 33 hindurch.

Der verlängerte Teil 21b des Zuluftkamins erstreckt sich in Längsrichtung ein wenig über den Abschlußkörper 33 der Heizeinrichtung hinaus, so daß oberhalb des Abschlußkörpers 33 eine Öffnung vorhanden ist, deren Durchmesser der Öffnung der Steckkappe 23 des in Fig. 1 gezeigten Zuluftkamins entspricht. In diese Öffnung ist ein Ventilelement 40 einsteckbar, das beim herkömmlichen Inhalationsgerät in die Öffnung der Steckkappe 23 des Zuluftkamins eingesteckt wird. Durch die Verlängerung des Zuluftkamins 21 über den Abschlußkörper 23 der Heizeinrichtung hinaus wird die Möglichkeit geschaffen, dieses Ventilelement auch bei einem Zuluftkamin einzusetzen, der gemäß diesem Ausführungsbeispiel mit der Heizeinrichtung 30 als Einheit ausgebildet ist. Im übrigen entspricht die Heizeinrichtung dieses Ausführungsbeispiels gemäß Fig. 4 dem Aufbau, der in Zusammenhang mit den Fig. 2 und 3 beschrieben wurde. Auch bei diesem Ausführungsbeispiel strömt die Umgebungsluft an den Rippen und dem Kernkörper des Wärmetauschers vorbei durch den Zuluftkamin und wird dabei erwärmt. Die dafür erforderlichen Temperaturen unterscheiden sich nicht von denen des ersten Ausführungsbeispiels.

Eine andere Möglichkeit für die Temperaturregelung bietet sich im Rahmen der Erfindung dahingehend, daß ein herkömmliches elektrisches Heizelement im Wärmetauscherteil der Heizeinrichtung vorgesehen wird und im Aerosolauslaßstutzen 22 ein Temperatursensor angeordnet wird, mit dessen Hilfe die Temperatur des Aerosols, das vom Patienten eingeatment wird, erfaßt wird. Aufgrund der erfaßten Temperatur kann dann die Heizleistung des herkömmlichen elektrischen Heizelements angepaßt werden. Dies ist insofern vorteilhaft, als es sich um einen kontrollierten Regelkreis handelt, der auf die Temperatur in einem Bereich zurückgreift, in dem sie zwischen 28 und 32°C gehalten werden soll, nämlich im Bereich des vom Patienten eingeatmeten Aerosols. Trotz des schaltungstechnisch höheren Aufwands kann diese Form der Temperatursteuerung in einigen Fällen vorteilhaft sein.

Um eine optimale Wärmeübertragung vom Heizelement zum Wärmetauscherteil der Heizeinrichtung zu erreichen, ist das Heizelement vorzugsweise an den Innendurchmesser des Kernkörpers des Wärmetauschers angepaßt und liegt mit seinen Aussenflächen fest an der Innenfläche des Kernkörpers an. Der Kernkörper ist vorzugsweise dünnwandig ausgestaltet, wohingegen die Rippen des Wärmetauschers zur besseren Wärmeleitung eine gewisse Dicke aufweisen.

## Patentansprüche

1. Vorrichtung zum Zerstäuben, Verteilen und Vermischen von flüssigen und pulverförmigen Substanzen mittels eines Druckgasstromes, insbesondere für die Erzeugung von Aerosolen für Inhalationszwecke, mit
- einer Zerstäubungsdüse (10), die in einem Zerstäubungsraum (29) angeordnet ist,
- einem Zuluftkamin (21) für den Eintritt von Zuluft in den Zerstäubungsraum (29), und
- einer Heizeinrichtung (30) zum Erwärmen der durch den Zuluftkamin angesaugten Umgebungsluft,
dadurch **gekennzeichnet,** daß
- die Heizeinrichtung (30) in dem Zuluftkamin (21) angeordnet ist und einen Wärmetauscher aus einem zylindrischen Kernkörper (31) und mehreren, insbesondere drei bis acht Rippen (32), die radial zum zylindrischen Kernkörper verlaufen, aufweist.

2. Vorrichtung nach Anspruch 1,
dadurch **gekennzeichnet,** daß
die Heizeinrichtung (30) ein elektrisches Heizelement (35) aufweist.

3. Vorrichtung nach Anspruch 2,
dadurch **gekennzeichnet,** daß
das elektrische Heizelement (35) ein PTC-Widerstand ist.

4. Vorrichtung nach einem der Ansprüche 1 bis 3,
dadurch **gekennzeichnet**, daß
die Rippen Auflageflächen (32a) aufweisen, die schräg zur Längsachse des zylindrischen Kernkörpers (31) verlaufen, auf denen die Heizeinrichtung im Bereich einer konischen Steckkappe (23) des Zuluftkamins (21) aufliegt.

5. Vorrichtung nach einem der Ansprüche 1 bis 4,
dadurch **gekennzeichnet,** daß
die Heizeinrichtung (30) einen Abschlußkörper (33) umfaßt.

6. Vorrichtung nach Anspruch 5,
dadurch **gekennzeichnet,** daß
sich der Wärmetauscher aus dem Zuluftkamin (21) heraus erstreckt und der Abschlußkörper (33) den Wärmetauscher allseitig derart überragt, daß eine unbeabsichtigte Berührung des Wärmetauschers verhindert wird.

7. Vorrichtung nach einem der Ansprüche 1 bis 6,
dadurch **gekennzeichnet,** daß
die Heizeinrichtung (30) mit dem Zuluftkamin (21) als Einheit ausgebildet ist.

8. Vorrichtung nach Anspruch 7,
dadurch **gekennzeichnet,** daß
der Zuluftkamin (21) einen zylindrischen Teil (21b) aufweist, der den herausragenden Teil des Wärmetauschers (31, 32) umgibt.

## Claims

1. Device for atomising, dispersing and mixing fluid or powdery substances by means of a stream of compressed gas, especially for the production of aerosols for inhalation purposes, having
- an atomising nozzle (10) which is arranged in an atomising space (29),
- an incoming air riser (21) for the entry of additional air into the atomising space (29), and
- a heating device (30) for heating the ambient air drawn in by the incoming air riser,
characterised in that
- the heating device (30) is arranged in the incoming air riser (21) and has a heat exchanger composed of a cylindrical core body (31) and a plurality of ribs (32), especially three to eight, which extend in radial manner with respect to the cylindrical core body.

2. Device according to claim 1,
characterised in that
the heating device (30) has an electric heating element (35).

3. Device according to claim 2,
characterised in that
the electric heating element (35) is a PTC resistance.

4. Device according to one of claims 1 to 3,
characterised in that
the ribs have supporting surfaces (32a) which run at a slope to the longitudinal axis of the cylindrical core body (31) on which the heating device is supported in the region of a conical plug cap (23) of the incoming air riser (21).

5. Device according to one of claims 1 to 4,
characterised in that
the heating device (30) comprises a closing body (33).

6. Device according to claim 5,
characterised in that
the heat exchanger extends out of the incoming air riser (21) and the closing body (33) projects on all sides over the heat exchanger in such a way that unintentional touching of the heat exchanger is prevented.

7. Device according to one of claims 1 to 6,
characterised in that
the heating device (30) is constructed as a unit with the incoming air riser (21).

8. Device according to claim 7,
characterised in that
the incoming air riser (21) has a cylindrical part (21b) which surrounds the projecting part of the heat exchanger (31, 32).

## Revendications

1. Dispositif pour pulvériser, distribuer et mélanger des substances liquides et des substances pulvérulentes au moyen d'un courant gazeux, notamment pour produire des aérosols destinés à être inhalés, avec
- une buse de pulvérisation (10) qui est disposée dans une chambre de pulvérisation (29),
- un conduit d'entrée d'air (21) pour l'admission d'air entrant dans la chambre de pulvérisation (29) et,
- un dispositif de chauffage (30) pour réchauffer l'air ambiant aspiré par le conduit d'entrée d'air,
caractérisé par le fait
- que le dispositif de chauffage (30) est disposé dans le conduit d'entrée d'air (21) et comporte un échangeur thermique qui est formé d'un corps central (31) cylindrique et de plusieurs nervures (32), en particulier de trois à huit nervures, qui s'étendent radialement à partir du corps central cylindrique.

2. Dispositif selon la revendication 1, caractérisé par le fait que le dispositif de chauffage (30) comporte un élément chauffant (35) électrique.

3. Dispositif selon la revendication 2, caractérisé par le fait que l'élément chauffant (35) électrique est une résistance à coefficient de température positif.

4. Dispositif selon l'une des revendications 1 à 3, caractérisé par le fait que les nervures présentent des surfaces d'appui (32a) qui s'étendent en biais par rapport à l'axe longitudinal du corps central (31) cylindrique et sur lesquelles le dispositif de chauffage repose dans la région d'un bouchon (23) enfichable conique du conduit d'entrée d'air (21).

5. Dispositif selon l'une des revendications 1 à 4, caractérisé par le fait que le dispositif de chauffage comprend un élément de fermeture (33).

6. Dispositif selon la revendication 5, caractérisé par le fait que l'échangeur thermique s'étend à l'extérieur du conduit d'entrée d'air (21) et que l'élément de fermeture (33) dépasse de tous les côtés par rapport à l'échangeur de chaleur de manière à empêcher tout contact involontaire avec ledit échangeur de chaleur.

7. Dispositif selon l'une des revendications 1 à 6, caractérisé par le fait que le dispositif de chauffage (30) forme une unité avec le conduit d'entrée d'air (21).

8. Dispositif selon la revendication 7, caractérisé par le fait que le conduit d'entrée d'air (21) comporte une partie (21b) cylindrique qui entoure la partie de l'échangeur thermique (31,32) qui fait saillie à l'extérieur.
